# EUROPEAN PATENT APPLICATION

(11) **EP 4 524 230 A1**
(43) Date of publication of application: **19.03.2025**
(21) Application number: 22940723.4
(22) Date of filing: 22.08.2022
(51) Int. Cl.: C12M 3/00, C12M 1/00, C12M 1/36

(54) **CULTURE VESSEL ACTIVE OSCILLATION-TYPE SLIDING TABLE MECHANISM AND SHAKER INCUBATOR**

(30) Priority: 06.05.2022 CN 202221064303 U
(71) Applicant: Innovel Intelligent Technology Co., Ltd., Suzhou, Jiangsu 215000 (CN); WuXi Biologics Ireland Limited, Dundalk, Co Louth A91 X56F (IE)
(72) Inventor: CHEN, Hao, Suzhou, Jiangsu 215000 (CN); YUAN, Yuanwei, Suzhou, Jiangsu 215000 (CN); TAN, Kee Wee, Shanghai 200131 (CN); DING, Jie, Shanghai 200131 (CN)
(74) Representative: Modiano, Gabriella Diana
(86) International application number: PCT/CN2022/113970
(87) International publication number: WO 2023/213029

(57) **Abstract**

The present application provides an culture vessel active-oscillation slide mechanism and a shaker incubator, which can improve the overall stiffness and stability of the slide mechanism to avoid the creation of lateral bending moment while ensuring the culture vessel in the shaker incubator to fully extend out, and can also simplify the assembly complexity of a stroke amplification mechanism and reduce the number of parts. The culture vessel active-oscillation slide mechanism comprises a slide power transmission device, a slide stroke amplification mechanism and a slide mounting plate. The slide power transmission device can convert a rotational motion of an output shaft of a slide drive motor into a linear displacement in a direction of width. The slide stroke amplification mechanism amplifies the stroke of the linear displacement of the slide power transmission device in the direction of width. Rigid multi-stage rails are mounted on two sides of the slide mounting plate in a direction of length of the incubator body. In the multi-stage rails are configured to, by means of stages of rails partially sliding out relative to each other, support a culture vessel carrier mounting plate that fully extends out after the stroke is amplified.

## Description

### Technical Field

The present invention relates to the technical field of biopharmaceutical production devices, and in particular to a culture vessel active-oscillation slide mechanism for use in an automated shaker incubator, and a shaker incubator having the culture vessel active-oscillation slide mechanism. The culture vessel active-oscillation slide mechanism enables a culture vessel carrier for holding culture vessels to automatically slide out and fully extend out so as to automatically perform operations such as sampling and adding a culture medium, and enables active oscillation for the culture vessel carrier after the culture vessel carrier is retracted.

### Background Art

Biopharmaceuticals are in a period of rapid development. macromolecular drugs such as antibodies are developing at a high speed. In the process of research and development and production of macromolecular drugs such as antibodies, a large number of shake flasks, shake tubes, well plates and other consumables for carrier and target product cultures are used, and shaker incubators are necessary production apparatus.

In a traditional shaker incubator, operations such as observation, testing, sampling, and adding of culture media are mainly carried out manually by the staff. The shaker incubator is difficult to interface with a newly developed automated transfer system, a liquid operation center, and intelligent scheduling and management software, may not be adapted to a rapid and automated research and development and production process of mass biological drugs, and is also unable to realize intelligent data acquisition, storage and transmission in the production process.

In order to achieve automated operation of the traditional shaker incubator, not only should the shaker incubator carrier be able to slide out automatically, but also the distance the carrier slides out must be greater than the width of the carrier itself, that is, the carrier needs to be able to fully extend out. Therefore, it is necessary to design a stroke amplification mechanism in it.

To this end, the inventors of the present invention has proposed an automatic sliding-out structure of a fully automatic shaker incubator carrier in Chinese utility model patent ZL 202120623168.3, the automatic sliding-out structure including a slide protective housing, wherein a second-layer sliding rail is mounted at the bottom carried by the culture carrier and located on a right side of a support wheel; an intermediate sliding rail fixing plate is mounted on a lower side of the second-layer sliding rail; a first-layer sliding rail is provided at the bottom of an inner wall of the slide protective housing and located on a lower side of the intermediate sliding rail fixing plate; a V-type wheel is mounted in the middle of a right side of the intermediate sliding rail fixing plate; and a wire rope is mounted on the V-type wheel. When in use, a reducer is started, and the reducer drives, through its output shaft, a gear to rotate so as to drive a rack to move forward and also drive the intermediate sliding rail fixing plate and the V-type wheel to move forward, and in turn drives the wire rope to rotate so as to drive the culture carrier to move forward relative to the V-type wheel, and in a culture unit, the culture carrier automatically slides in or slides out to seamlessly interface with an automatic transfer system. In addition, the wire rope can achieve a secondary amplification of stroke, such that the automatic sliding-out mechanism can slide out over a long distance within a limited space, so as to enable the culture carrier to completely slide out of the culture unit.

In simple terms, in Chinese utility model patent ZL 202120623168.3, two layers of sliding rails (the first-layer sliding rail and the second-layer sliding rail) are used to achieve a secondary amplification of stroke, which can ideally enable culture vessels in the shaker incubator to fully extend out. However, in such a structure, due to the secondary extension of the second-layer sliding rail relative to the first-layer sliding rail, the overall stiffness and stability of structure significantly decrease after the full extension, which may cause deformation or swaying (there is lateral bending moment) and thus cause the position of the culture carrier for holding the culture vessels to deviate from the target position. In this case, during the operation on the culture carrier using a manipulator of an automation system, the automation system has to determine whether and how the culture carrier deviates from the target position every time, resulting in significant decrease of automation efficiency, and may even be impossible to continue the automated operation when the culture carrier is severely deformed.

In addition, in the aforementioned method of using the two layers of sliding rails to achieve the secondary amplification of stroke, there are still problems such as a large number of parts and complex assembly of equipment.

Therefore, how to improve the overall stiffness and stability of the slide mechanism to avoid the creation of lateral bending moment while ensuring the culture vessels in the shaker incubator to ideally fully extend out and how to also simplify the assembly complexity of a stroke amplification mechanism and reduce the number of parts have become a technical problem that needs to be solved urgently.

### Disclosure of the Invention

### Technical problems to be solved by the invention

In order to solve the above technical problems, the objective of the present invention is to provide a culture vessel active-oscillation slide mechanism and a shaker incubator having the culture vessel active-oscillation slide mechanism, in order to solve the problems mentioned in the background art.

### Technical solutions employed to solve the technical problems

In order to achieve the above objective, the present invention provides a culture vessel active-oscillation slide mechanism. The culture vessel active-oscillation slide mechanism is arranged inside an incubator body and includes: a slide protective housing, which protects, in a circumferential direction, components of the culture vessel active-oscillation slide mechanism arranged inside the slide protective housing; and a slide drive motor, which is configured to enable a culture vessel carrier mounting plate of the culture vessel active-oscillation slide mechanism to slide out or slide in relative to the incubator body. The culture vessel active-oscillation slide mechanism further includes: a slide power transmission device, which is connected, via a coupling, to an output shaft of the slide drive motor configured to output rotational power and can convert the rotational motion of the output shaft into a linear displacement in a direction of width of the incubator body; a slide stroke amplification mechanism, which is fixed to the slide power transmission device and configured to amplify the stroke of the linear displacement of the slide power transmission device in the direction of width; and a slide mounting plate, to which a portion of the slide power transmission device and the slide drive motor are fixedly mounted, rigid multi-stage rails being mounted on two sides of the slide mounting plate in a direction of length of the incubator body, wherein the multi-stage rails are configured to, by means of stages of rails partially sliding out relative to each other, support the culture vessel carrier mounting plate that fully extends out after the stroke is amplified.

According to the configuration as described above, the culture vessel active-oscillation slide mechanism of the present invention has the following beneficial effects compared to the prior art.

1. The culture vessel active-oscillation slide mechanism arranged inside the incubator body is used to enable the culture vessel carrier mounting plate (more accurately, the culture vessel carrier assembly mounted thereon) to automatically slide in or slide out relative to the incubator body to seamlessly interface with the automatic transfer system.

2. In the culture vessel active-oscillation slide mechanism, the slide stroke amplification mechanism in addition to the multi-stage rails is used to ensure the culture vessel carrier mounting plate (the culture vessel carrier assembly in the shaker incubator) of the culture vessel active-oscillation slide mechanism to ideally fully extend out, so that the culture vessel active-oscillation slide mechanism can slide out over a long distance in a limited space, so as to enable the culture vessel carrier mounting plate (more accurately, the culture vessel carrier assembly mounted thereon) to completely slide out of the incubator body of the shaker incubator, which is convenient for the automated operation of an external truss robot.

3. Since the multi-stage rails are not used for stroke amplification but are only used to support the culture vessel carrier mounting plate after the stroke is amplified, compared to a multi-stage rail in which stages of rails have to completely slide out relative to each other as much as possible for stroke amplification, the multi-stage rails can, by means of the stages of rails partially sliding out relative to each other, improve the overall stiffness and stability of the slide mechanism to avoid the creation of lateral bending moment, and can also avoid the situation that the fully extended culture vessel carrier mounting plate (more accurately, the culture vessel carrier assembly mounted thereon) is supported by a sliding rail that is provided as a cantilever.

Preferably, the slide power transmission device is of a lead screw structure that has a lead screw and a lead screw nut screwed onto the lead screw, the lead screw is connected to the output shaft of the slide drive motor via the coupling, and the lead screw of the slide power transmission device and the coupling are fixedly mounted to the slide mounting plate.

According to the configuration as described above, the conversion of the rotational motion of the output shaft into the linear displacement in the direction of width of the incubator body can be achieved by a simple structure.

Preferably, the slide stroke amplification mechanism is a multi-connecting-rod mechanism composed of a first multi-section connecting rod and a second multi-section connecting rod, and has a fixing portion, a fulcrum portion and a stroke amplification portion. More preferably, the slide power transmission device is arranged, along the direction of width of the incubator body, at a middle position of the slide mounting plate in the direction of length, and the fixing portion, the fulcrum portion and the stroke amplification portion are in a straight line.

According to the configuration as described above, the culture vessel carrier mounting plate can reliably slide in or slide out linearly along the direction of width.

In this case, further preferably, a distance between the stroke amplification portion and the fulcrum portion in the direction of width is greater than or equal to a distance between the fulcrum portion and the fixing portion in the direction of width, such that the slide power transmission device enables, with a small stroke of linear displacement, the culture vessel carrier mounting plate to rapidly slide in or slide out and enables the stroke of linear displacement to be doubled or more.

In addition, as a specific structure, the first multi-section connecting rod and the second multi-section connecting rod have the same structure in which a first connecting rod, a second connecting rod and a third connecting rod are connected in sequence by fasteners to form a three-section connecting rod, wherein one ends of the first connecting rods are connected to each other to form a fixing portion, the third connecting rods are connected to each other to form a stroke amplification portion, and the second connecting rods are connected to each other in the middle to form a fulcrum portion.

In this way, compared to the existing structure in which two layers of sliding rails (the first-layer sliding rail and the second-layer sliding rail) are used to achieve secondary amplification of stroke, it is possible to simplify the assembly complexity of the stroke amplification mechanism and reduce the number of parts.

In this case, preferably, in the direction of length, a connecting portion between the first connecting rod and the second connecting rod and a connecting portion between the third connecting rod and the second connecting rod of each of the first multi-section connecting rod and the second multi-section connecting rod are close to the multi-stage rails on the two sides of the slide mounting plate.

According to the configuration as described above, it is possible to effectively use the space inside the slide protective housing in the direction of length to avoid the slide stroke amplification mechanism being of a large size in the direction of width.

In addition, as a specific structure, the multi-stage rails located inside the slide protective housing and on the two sides of the slide mounting plate each have a fixed rail and two stages of sliding rails, namely a first-stage sliding rail and a second-stage sliding rail, and as the culture vessel carrier mounting plate extends out, the culture vessel carrier mounting plate is supported by the second-stage sliding rail in such a manner that the first-stage sliding rail partially slides out relative to the fixed rail and the second-stage sliding rail partially slides out relative to the first-stage sliding rail. Also, more preferably, the first-stage sliding rail slides out no more than half relative to the fixed rail, and the second-stage sliding rail slides out no more than half relative to the first-stage sliding rail.

According to the configuration as described above, it is possible to avoid the situation that the fully extended culture vessel carrier mounting plate (or the culture vessel carrier assembly mounted thereon) is supported by the sliding rail that is provided as a cantilever, which, compared to the existing method of achieving stroke amplification using the two layers of sliding rails (the first-layer sliding rail and the second-layer sliding rail) in which the second-layer sliding rail has to slide out as much as possible relative to the first-layer sliding rail, can improve the overall stiffness and stability of the slide mechanism more reliably to avoid the creation of lateral bending moment while ensuring the culture vessel carrier mounting plate (or the culture vessel carrier assembly mounted thereon) to ideally fully extend out.

In addition, the culture vessel active-oscillation slide mechanism of the present invention further includes a main oscillating motor. The main oscillating motor is fixedly mounted to the slide mounting plate and configured to enable the culture vessel active-oscillation slide mechanism to actively oscillate. More specifically, an oscillation driving shaft portion and a plurality of oscillation driven shaft portions are provided below the slide mounting plate. Each of the oscillation driving shaft portion and the oscillation driven shaft portions includes: a bearing pedestal fixed below the slide mounting plate; a rolling bearing arranged in the respective bearing pedestal; and an eccentric shaft that is eccentric relative to the respective rolling bearing. The eccentric shaft of the oscillation driving shaft portion is connected to the eccentric shaft of each of the oscillation driven shaft portions via a belt pulley, and the eccentric shaft of the oscillation driving shaft portion is connected to an output shaft of the main oscillating motor.

According to the configuration as described above, the main oscillating motor drives the eccentric shaft of the oscillation driving shaft portion to oscillate relative to the bearing pedestal and the rolling bearing and drives, via the belt pulley, the eccentric shafts of other oscillation driven shaft portions to oscillate along with the bearing pedestal 573a and the rolling bearing, which can achieve more uniform oscillation of the culture vessel active-oscillation slide mechanism and in turn of the culture vessels of the culture vessel carrier assembly.

The present invention further provides a shaker incubator provided with the aforementioned culture vessel active-oscillation slide mechanism. The culture vessel active-oscillation slide mechanism enables the culture vessel carrier assembly to automatically slide out relative to the incubator body and fully extend out after the automatic door of the automatic seal door assembly is opened, and enables the culture vessel carrier assembly to automatically slide into the incubator body before the automatic door of the automatic seal door assembly is closed and to actively oscillate after the automatic door of the automatic seal door assembly is closed. Also, the automatic seal door assembly and the culture vessel active-oscillation slide mechanism are provided as the automated components.

In addition, according to the configuration as described above, the shaker incubator of the present invention has the following beneficial effects compared to the prior art.

1. With the automation design of the shaker incubator, in particular, the aforementioned culture vessel active-oscillation slide mechanism and the automatic seal door assembly, it is possible to realize fully automatic operations such as automatic door opening, automatic sliding out of the carrying plate, automatic sampling and placing, automatic sliding in of the carrying plate, automatic door closing, and start and stop of active oscillation, thereby reducing labor costs and reducing human operation errors and risks.

2. With the overall design of the automated shaker incubator, integration with an automatic production system is enabled, which allows for large-scale deployment and a significant increase in production capacity and throughput.

3. With the overall design of the automated shaker incubator, integration with an information system is enabled, so as to allow for automatic uploading and storage of various types of data, which facilitates product tracking and also ensures the integrity and traceability of data.

### Effects of the invention

According to the configuration as described above, the culture vessel active-oscillation slide mechanism and the shaker incubator having the culture vessel active-oscillation slide mechanism of the present invention can improve the overall stiffness and stability of the slide mechanism to avoid the creation of lateral bending moment while ensuring the culture vessels in the shaker incubator to ideally fully extend out, and can also simplify the assembly complexity of the stroke amplification mechanism and reduce the number of parts.

### Brief Description of the Drawings

FIG. 1 is a schematic perspective view schematically showing the overall structure and internal structure of an automated shaker incubator of the present invention.
FIG. 2 is a perspective view of the culture vessel active-oscillation slide mechanism of the present invention from one perspective from an oblique top view.
FIG. 3 is a perspective view of the culture vessel active-oscillation slide mechanism of the present invention from another perspective from an oblique bottom view.
FIG. 4 is a transverse cross-sectional view of the culture vessel active-oscillation slide mechanism of the present invention cut along a direction of length (a left-right direction).
FIG. 5 is a longitudinal cross-sectional view of the culture vessel active-oscillation slide mechanism of the present invention cut along a direction of width (a front-rear direction).
FIG. 6 is a schematic diagram of initial state of the culture vessel active-oscillation slide mechanism of the present invention in a retracted state.
FIG. 7 is a schematic diagram of slide-out state of the culture vessel active-oscillation slide mechanism of the present invention in a fully extended state.

### (List of reference signs)

- 10: Shaker incubator;
- 100: Incubator body;
- 110: Automatic seal door assembly;
- 111: Automatic door;
- 112: Observation window;
- 120: Supporting leg;
- 200: Apparatus illumination/purification/disinfection unit;
- 300: Intelligent integrated control system unit;
- 400: Culture vessel carrier assembly;
- 410: Carrying plate;
- 420: Culture vessel;
- 500: Culture vessel active-oscillation slide mechanism;
- 510: Slide protective housing;
- 520: Main oscillating motor;
- 530: Slide drive motor;
- 531: Output shaft;
- 540: Slide power transmission device;
- 541: Lead screw;
- 542: Lead screw nut;
- 543: Coupling;
- 550: Slide stroke amplification mechanism;
- 551: Carrier mounting plate connection portion;
- 552: First multi-section connecting rod;
- 552a: First connecting rod of first multi-section connecting rod;
- 552b: Second connecting rod of first multi-section connecting rod;
- 552c: Third connecting rod of first multi-section connecting rod;
- 553: Second multi-section connecting rod;
- 553a: First connecting rod of second multi-section connecting rod;
- 553b: Second connecting rod of second multi-section connecting rod;
- 553c: Third connecting rod of second multi-section connecting rod;
- 560: Culture vessel carrier mounting plate;
- 561: Culture vessel carrier positioning pin;
- 570: Slide mounting plate;
- 571: Multi-stage rail;
- 571a: Fixed rail;
- 571b: First-stage sliding rail;
- 571c: Second-stage sliding rail;
- 572: Oscillation driving shaft portion;
- 572a: Bearing pedestal of oscillation driving shaft portion;
- 572b: Rolling bearing of oscillation driving shaft portion;
- 572c: Eccentric shaft of oscillation driving shaft portion;
- 573: Oscillation driven shaft portion;
- 573a: Bearing pedestal of oscillation driven shaft portion;
- 573b: Rolling bearing of oscillation driven shaft portion;
- 573c: Eccentric shaft of oscillation driven shaft portion.

### Detailed Description of Embodiments

The technical solutions of the embodiments of the present invention will be clearly and completely described below with reference to the drawings of the embodiments of the present invention. Obviously, the embodiments described are merely some, rather than all, of the embodiments of the present utility model, and based on the embodiments of the present invention, all other embodiments obtained by those skilled in the art without involving any inventive effort will fall within the scope of protection of the present invention.

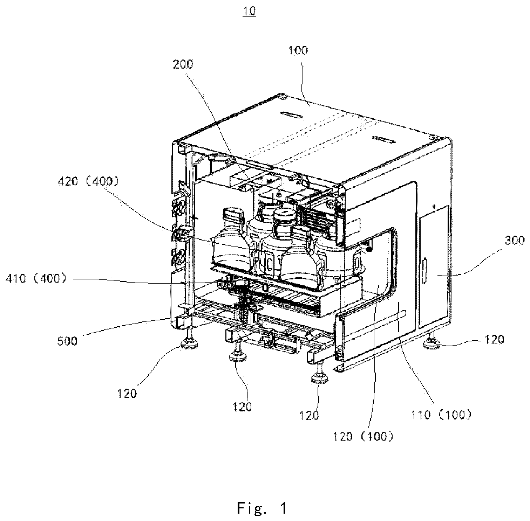

First, an automated shaker incubator 10 of the present invention is described in detail with reference to FIG. 1.

The automated shaker incubator 10 of the present invention shown in FIG. 1 includes an incubator body 100. An automatic seal door assembly 110 is provided on a front side of the incubator body 100. The automatic seal door assembly 110 includes: an automatic door 111 capable of being opened and closed automatically based on system control; and, for example, a door opening and closing drive motor, a driving gear, and a driven gear (not shown), which are configured to implement automatic opening and closing of the automatic door 111.

In this embodiment, an observation window 112 is arranged on a front side of the automatic door 111. The automatic door 111 is connected to the incubator body 100 of the shaker incubator 10, for example, by means of engagement. Furthermore, in this embodiment, although not shown in the figures, the driving gear is arranged at a drive end of the door opening and closing drive motor, and the driven gear is connected to the automatic door 111. The driving gear is connected to the driven gear by means of engagement. However, the present invention is not limited thereto. The automatic opening and closing of the automatic door 111 may be realized, for example, by means of transmission between the driving gear, the driven gear and a belt, or by means of extension and retraction of a connecting rod.

In addition, a visual operation portion (not shown) is provided on the front side of the incubator body 100 (the right side in FIG. 1). The visual operation portion is, for example, a touch screen that can implement both mode operation and visual current-state confirmation. However, the present invention is not limited thereto, and the visual operation portion may also be a combination of a button that realizes only mode operation and a display screen that realizes only visual current-state confirmation.

In addition, multiple (for example, six) supporting legs 120 are provided at the bottom of the shaker incubator 10. Due to the limitation of the perspective, FIG. 1 only shows four supporting legs 120, but the present invention is not limited thereto. As long as the shaker incubator 10 can ensure stability during operation, it is also possible to provide no supporting leg or to provide a different number of supporting legs from six.

In addition, as shown in FIG. 1, the incubator body 100 of the shaker incubator 10 includes therein: an apparatus illumination/purification/disinfection unit 200, the apparatus illumination/purification/disinfection unit 200 having an ultraviolet lamp and a fluorescent lamp for illuminating, purifying and disinfecting the interior of the incubator body 100; an intelligent integrated control system unit 300, the intelligent integrated control system unit 300 having a PLC controller, a data storage chip, a data transmission device, and a remote control device and being configured to control actions of various automated components inside the incubator body 100; an environment control unit (not shown), which has a temperature sensor, a humidity sensor and a light sensor to control environmental parameters such as temperature, humidity and light of the interior of the incubator body 100; a shake flask/shake tube/well plate carrier assembly (also referred to as "culture vessel carrier assembly 400"), which has a carrying plate 410 and a shake flask/shake tube/well plate (also referred to as "culture vessel 420") carried on the carrying plate 410; and a culture vessel active-oscillation slide mechanism 500. The culture vessel active-oscillation slide mechanism 500 enables the culture vessel carrier assembly 400 to automatically slide out relative to the incubator body 100 and fully extend out after the automatic door 111 of the automatic seal door assembly 110 is opened, so that a robotic arm of an external truss robot can perform automated operations on the culture vessels 420 on the culture vessel carrier assembly 400, and the culture vessel active-oscillation slide mechanism 500 enables the culture vessel carrier assembly 400 to automatically slide into the incubator body 100 before the automatic door 111 of the automatic seal door assembly 110 is closed and to actively oscillate after the automatic door 111 of the automatic seal door assembly 110 is closed, and also serves as a shaker mechanism that shakes the culture vessel carrier assembly 400.

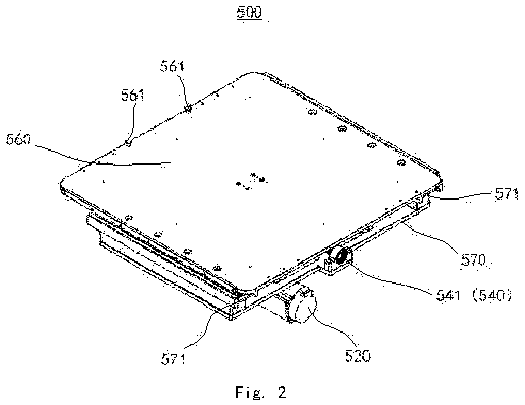

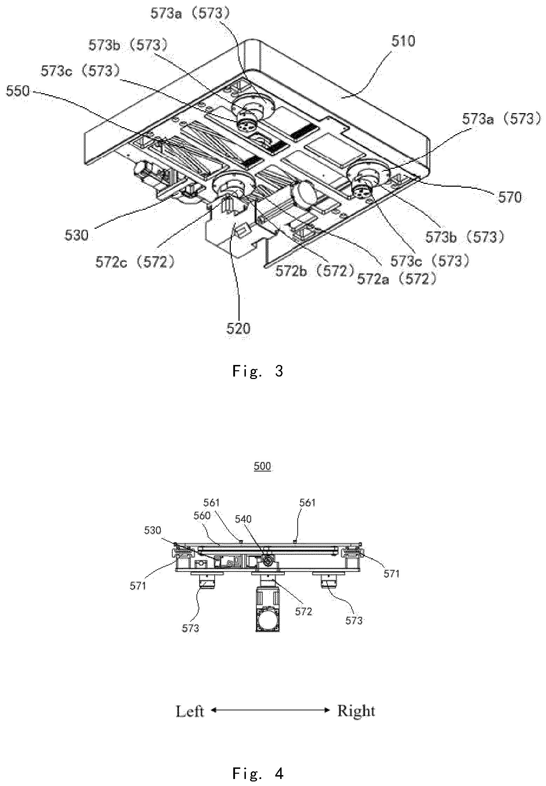

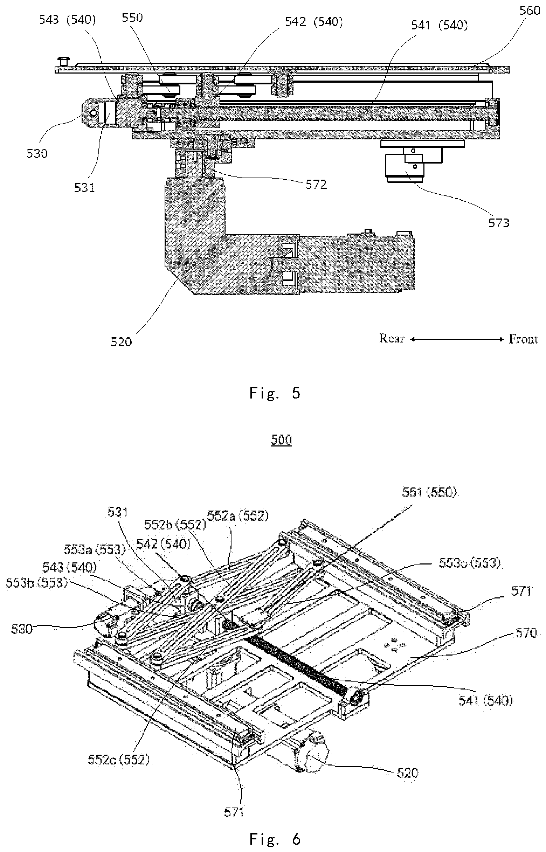

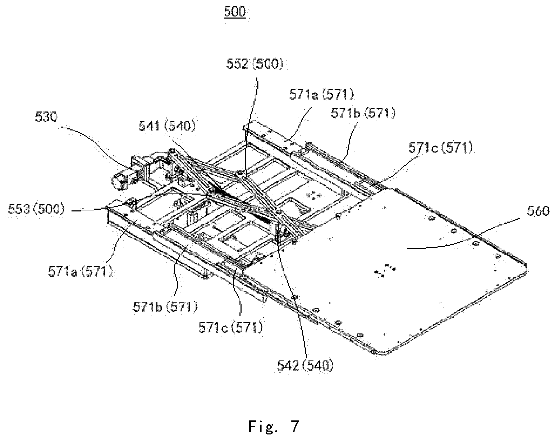

The structure of the culture vessel active-oscillation slide mechanism 500 of the present invention inside the incubator body 100 will be described in detail below with reference to FIGS. 2 to 7. FIG. 2 is a perspective view of the culture vessel active-oscillation slide mechanism 500 of the present invention from one perspective from an oblique top view, FIG. 3 is a perspective view of the culture vessel active-oscillation slide mechanism 500 of the present invention from another perspective from an oblique bottom view, FIG. 4 is a transverse cross-sectional view of the culture vessel active-oscillation slide mechanism 500 of the present invention cut along a direction of length (a left-right direction), FIG. 5 is a longitudinal cross-sectional view of the culture vessel active-oscillation slide mechanism 500 of the present invention cut along a direction of width (a front-rear direction), FIG. 6 is a schematic diagram of initial state of the culture vessel active-oscillation slide mechanism 500 of the present invention in a retracted state, and FIG. 7 is a schematic diagram of slide-out state of the culture vessel active-oscillation slide mechanism 500 of the present invention in a fully extended state.

As shown in FIG. 3, the culture vessel active-oscillation slide mechanism 500 inside the incubator body 100 of the fully automatic shaker incubator 10 according to the embodiments of the present invention includes a slide protective housing 510. The slide protective housing 510 protects, in a circumferential direction, the components of the culture vessel active-oscillation slide mechanism 500 arranged therein.

In order to better illustrate the components of the culture vessel active-oscillation slide mechanism 500, the slide protective housing 510 is not shown in FIGS. 2, 4 and 5.

As shown in FIGS. 2 to 5, the culture vessel active-oscillation slide mechanism 500 includes: a main oscillating motor 520, the main oscillating motor 520 being configured to actively oscillate the culture vessel active-oscillation slide mechanism 500 (more specifically, the slide mounting plate 570 and the culture vessel carrier mounting plate 560 described later); a slide drive motor 530, the slide drive motor 530 being configured to enable the culture vessel active-oscillation slide mechanism 500 (more specifically, the culture vessel carrier mounting plate 560 described later) to slide out or slide in relative to the incubator body 100, and having an output shaft 531 (see FIG. 5) that outputs rotational power; a slide power transmission device 540, the slide power transmission device 540 being arranged, along the front-rear direction (the direction of width), at the middle in the left-right direction (the direction of length), being connected to the output shaft 531 of the slide drive motor 530 via a coupling 543, and being capable of converting the rotational motion of the output shaft 531 into a linear displacement in the front-rear direction (the direction of width); a slide stroke amplification mechanism 550, the slide stroke amplification mechanism 550 being fixed to the slide power transmission device 540 and configured to amplify the stroke of the linear displacement of the slide power transmission device 540 in the front-rear direction (the direction of width), and a carrier mounting plate connection portion 551 for mounting a culture vessel carrier mounting plate 560 described later being fixed to the slide stroke amplification mechanism 550; and a slide mounting plate 570, the main oscillating motor 520, the slide drive motor 530 and (a portion of) the slide power transmission device 540 being fixedly mounted on the slide mounting plate 570, and rigid multi-stage rails 571 being mounted on two sides of the slide mounting plate 570 in the left-right direction (the direction of length), wherein the multi-stage rails 571 are configured to, by means of the stages of rails partially sliding out relative to each other, support the culture vessel carrier mounting plate 560 that fully extends out after the stroke is amplified. In addition, as shown in FIG. 3, an oscillation driving shaft portion 572 and a plurality of oscillation driven shaft portions 573 are provided below the slide mounting plate 570. Each of the oscillation driving shaft portion 572 and the oscillation driven shaft portions 573 includes: a bearing pedestal 572a, 573a fixed below the slide mounting plate 570; a rolling bearing 572b, 573b arranged in the respective bearing pedestal 572a, 573a; and an eccentric shafts 572c, 573c that is eccentric relative to the respective rolling bearing 572b, 573b. The eccentric shafts 572c, 573c of the oscillation driving shaft portion 572 and the oscillation driven shaft portion 573 are connected to each other via a belt pulley that is not shown in the figure, and the eccentric shaft 572c of the oscillation driving shaft portion 572 is connected to the output shaft of the main oscillating motor 520. The main oscillating motor 520 drives the eccentric shaft 572c of the oscillation driving shaft portion 572 to oscillate relative to the bearing pedestal 572a and the rolling bearing 572b and drives, via the belt pulley, the eccentric shafts 573c of other oscillation driven shaft portions 573 to oscillate along with the bearing pedestal 573a and the rolling bearing 573b, which can achieve more uniform oscillation of the culture vessel active-oscillation slide mechanism 500 and in turn of the culture vessels 420 of the culture vessel carrier assembly 400.

In the embodiments of the present invention, as a specific example, the slide power transmission device 540 may be of, for example, a lead screw structure that has a lead screw 541 and a lead screw nut 542 screwed onto the lead screw 541, as shown in FIGS. 4 to 7. The lead screw 541 is connected to the output shaft 531 of the slide drive motor 530 via the coupling 543. The lead screw 541 of the slide power transmission device 540 and the coupling 543 are fixedly mounted to the slide mounting plate 570.

As a specific example that can be used cooperatively with the aforementioned lead screw structure having the lead screw 541 and the lead screw nut 542, the slide stroke amplification mechanism 550 may be, for example, a multi-connecting-rod mechanism composed of a first multi-section connecting rod 552 and a second multi-section connecting rod 553. As shown in FIGS. 6 and 7, the first multi-section connecting rod 552 is, for example, a three-section connecting rod composed of a first connecting rod 552a, a second connecting rod 552b and a third connecting rod 552c connected in sequence by fasteners (such as bolts), and the second multi-section connecting rod 553 is, for example, a three-section connecting rod composed of a first connecting rod 553a, a second connecting rod 553b and a third connecting rod 553c connected in sequence by fasteners (such as bolts). In addition, one end of the first connecting rod 552a of the first multi-section connecting rod 552 and one end of the first connecting rod 553a of the second multi-section connecting rod 553 are connected to each other by a fastener (such as a bolt) and fixed to the coupling 543. Since the coupling 543 is fixedly mounted to the slide mounting plate 570, one end of the slide stroke amplification mechanism 550 serves as a fixing portion that does not displace. One end of the third connecting rod 552c of the first multi-section connecting rod 552 and one end of the third connecting rod 553c of the second multi-section connecting rod 553 are connected to each other by a fastener (such as a bolt) to form a stroke amplification portion for fixed mounting of the culture vessel carrier mounting plate 560. The second connecting rod 552b of the first multi-section connecting rod 552 and the second connecting rod 553b of the second multi-section connecting rod 553 are connected to each other in the middle by a fastener (such as a bolt) and fixed to the lead screw nut 542 to form a fulcrum portion that can perform linear displacement in the front-rear direction and can serve as a rotating fulcrum that enables the entire slide stroke amplification mechanism 550 to amplify the stroke of displacement. In addition, in this example, preferably, the fixing portion, the fulcrum portion and the stroke amplification portion are in a straight line, which reliably enables the culture vessel carrier mounting plate 560 to linearly slide in or slide out in the front-rear direction (the direction of width). In addition, as shown in FIGS. 6 and 7, further preferably, a distance between the stroke amplification portion and the fulcrum portion in the front-rear direction (the direction of width) is greater than or equal to a distance between the fulcrum portion and the fixing portion in the front-rear direction (the direction of width), such that the slide power transmission device 540 enables, with a small stroke of linear displacement, the culture vessel carrier mounting plate 560 to rapidly slide in or slide out. On this basis, more preferably, the connecting portions between the respective first connecting rods 552a, 553a and second connecting rods 552b, 553b and the connecting portions between the respective third connecting rods 552c, 553c and second connecting rods 552b, 553b of the first multi-section connecting rod 552 and the second multi-section connecting rod 553 are close to the multi-stage rails 571 on two sides of the slide mounting plate 570 in the left-right direction (the direction of length), which can effectively use the space inside the slide protective housing 510 in the left-right direction (the direction of length) to avoid the slide stroke amplification mechanism 550 being of a large size in the front-rear direction (the direction of width).

The culture vessel carrier mounting plate 560 is used for placing the carrying plate 410 of the culture vessel carrier assembly 400. More specifically, multiple (two illustrated in FIG. 2) culture vessel carrier positioning pins 561 are provided on one side (e.g., the rear side in FIG. 2) of the culture vessel carrier mounting plate 560 in the front-rear direction (the direction of width) in, for example, a left-right symmetrical manner. In addition, although not shown, corresponding multiple culture vessel carrier positioning holes are formed in the culture vessel carrier assembly 400. By aligning the culture vessel carrier positioning pins 561 of the culture vessel active-oscillation slide mechanism 500 with the culture vessel carrier positioning holes of the culture vessel carrier assembly 400, the culture vessel carrier assembly 400 (more specifically, the carrying plate 410) can be accurately positioned and arranged on the culture vessel carrier mounting plate 560 of the culture vessel active-oscillation slide mechanism 500. In this way, as the culture vessel active-oscillation slide mechanism 500 slides out or slides in relative to the incubator body 100 when the automatic door 111 is opened or closed, the culture vessel carrier assembly 400 (more specifically, the carrying plate 410 and the culture vessel 420) positioned and arranged on the culture vessel carrier mounting plate 560 of the culture vessel active-oscillation slide mechanism 500 can also fully extend out or fully retract relative to the incubator body 100.

In addition, the multi-stage rails 571 located inside the slide protective housing 510 and on the two sides of the slide mounting plate 570 each have a fixed rail 571a and two stages of sliding rails, namely a first-stage sliding rail 571b and a second-stage sliding rail 571c, and as the culture vessel carrier mounting plate 560 extends out, the culture vessel carrier mounting plate 560 is supported by the second-stage sliding rail 571c in such a manner that the first-stage sliding rail 571b partially slides out relative to the fixed rail 571a and the second-stage sliding rail 571c partially slides out relative to the first-stage sliding rail 571b.

In this case, preferably, the first-stage sliding rail 571b slides out no more than half relative to the fixed rail 571a, and the second-stage sliding rail 571c slides out no more than half relative to the first-stage sliding rail 571b, which can avoid the situation that the fully extended culture vessel carrier mounting plate 560 (the culture vessel carrier assembly 400) is supported by the sliding rail that is provided as a cantilever, which, compared to the solution of Chinese utility model patent ZL 202120623168.3 of achieving stroke amplification using two layers of sliding rails (a first-layer sliding rail and a second-layer sliding rail) in which the second-layer sliding rail has to slide out as much as possible relative to the first-layer sliding rail, can improve the overall stiffness and stability of the slide mechanism to avoid the creation of lateral bending moment while ensuring the culture vessel carrier assembly 400 in the shaker incubator 10 to ideally fully extend out.

Referring to FIGS. 6 and 7, the sliding between the culture vessel active-oscillation slide mechanism 500 in a retracted state shown in FIG. 6 and the culture vessel active-oscillation slide mechanism 500 in a fully extended state shown in FIG. 7 will be described blow.

More specifically, when sliding from the retracted state shown in FIG. 6 to the fully extended state shown in FIG. 7, the slide drive motor 530 is started, the output shaft 531 of the slide drive motor 530 rotates in one direction, and the lead screw 541 is also driven via the coupling 543 to rotate in one direction, such that the lead screw nut 542 screwed onto the lead screw 541 can move forward relative to the lead screw 541. At this time, the fulcrum portion of the slide stroke amplification mechanism 550 fixed to the lead screw nut 542 moves forward, such that the respective second connecting rods 552b, 553b of the first multi-section connecting rod 552 and the second multi-section connecting rod 553 rotate in such a manner that the angles between the second connecting rods and the first connecting rods 552a, 553a are increased, then the angles between the second connecting rods 552b, 553b and the third connecting rods 552c, 553c are increased, and thus the distance between the stroke amplification portion and the fulcrum portion is increased, that is, the stroke of displacement of the stroke amplification portion relative to the fixing portion is amplified, so as to enable the culture vessel carrier mounting plate 560 to fully extend out.

On the contrary, when sliding from the fully extended state shown in FIG. 7 to the retracted state shown in FIG. 6, the slide drive motor 530 is started to drive the output shaft 531 of the slide drive motor 530 to rotate in the opposite direction, such that the lead screw nut 542 can move backward relative to the lead screw 541. At this time, the fulcrum portion of the slide stroke amplification mechanism 550 moves backward, such that the respective second connecting rods 552b, 553b of the first multi-section connecting rod 552 and the second multi-section connecting rod 553 rotate in such a manner that the angles between the second connecting rods and the first connecting rods 552a, 553a and between the second connecting rods and the third connecting rods 552c, 553c are reduced, and thus the distance between the stroke amplification portion and the fulcrum portion is reduced, that is, the effect of the amplification of the stroke of displacement brought about by the stroke amplification portion is completely eliminated, so as to enable the culture vessel carrier mounting plate 560 to be fully retracted. In addition, after being fully retracted, the main oscillating motor 520 arranged below the slide mounting plate 570 can achieve uniform active oscillation of the culture vessel active-oscillation slide mechanism 500 and in turn of the culture vessel 420 of the culture vessel carrier assembly 400.

Although the embodiments of the present invention have been shown and described, it can be understood by those of ordinary skill in the art that various changes, modifications, substitutions and variations can be made to these embodiments without departing from the principles and spirit of the present invention, and the scope of the present invention is defined by the appended claims and their equivalents.

In the embodiments of the present invention, the slide power transmission device 540 is described in detail by taking a lead screw structure as an example, but it will be appreciated by those skilled in the art that the slide power transmission device 540 of the present invention is not limited to the aforementioned lead screw structure, but may be any suitable structure that can convert the rotational motion of the output shaft 531 into a linear displacement in the front-rear direction (the direction of width).

In addition, in the embodiments of the present invention, the slide stroke amplification mechanism 550 is described by taking the multi-connecting-rod mechanism composed of the first multi-section connecting rod 552 and the second multi-section connecting rod 553 as an example, but the slide stroke amplification mechanism 550 of the present invention is not limited thereto, and may be any other suitable structure, as long as it can be fixed to the slide power transmission device 540 and achieve the amplification of the stroke of linear displacement of the slide power transmission device 540 in the front-rear direction (the direction of width) in a manner other than a multi-layer sliding rail. In addition, the example of the multi-connecting-rod mechanism composed of the first multi-section connecting rod 552 and the second multi-section connecting rod 553 is described by taking the first multi-section connecting rod 552 and the second multi-section connecting rod 553 having the same structure as an example, but it should be appreciated by those skilled in the art that the first multi-section connecting rod 552 and the second multi-section connecting rod 553 may also have different structures, as long as the same or equivalent technical effects can be achieved.

In addition, in the embodiments of the present invention, the example of the multi-stage rail 571 having the fixed rail 571a and the two stages of sliding rails, namely the first-stage sliding rail 571b and the second-stage sliding rail 571c, is taken for description, but the present invention is not limited thereto. As long as the culture vessel active-oscillation slide mechanism 500 as a whole is not bulky due to a large size, it can also have more stages of sliding rails, which can reduce the sliding amount of each sliding rail relative to other rails so as to improve the supporting rigidity of the culture vessel carrier mounting plate 560 to avoid the creation of lateral bending moment.

## Claims

1. A culture vessel active-oscillation slide mechanism (500), **characterized in that** the culture vessel active-oscillation slide mechanism (500) is arranged inside an incubator body (100) and comprises:
a slide protective housing (510), the slide protective housing (510) protecting, in a circumferential direction, components of the culture vessel active-oscillation slide mechanism (500) arranged inside the slide protective housing; and
a slide drive motor (530), the slide drive motor (530) being configured to enable a culture vessel carrier mounting plate (560) of the culture vessel active-oscillation slide mechanism (500) to slide out or slide in relative to the incubator body (100),
**characterized in that** the culture vessel active-oscillation slide mechanism (500) further comprises:
a slide power transmission device (540), the slide power transmission device (540) being connected, via a coupling (543), to an output shaft (531) of the slide drive motor (530) configured to output rotational power, and being capable of converting the rotational motion of the output shaft (531) into a linear displacement in a direction of width of the incubator body (100);
a slide stroke amplification mechanism (550), the slide stroke amplification mechanism (550) being fixed to the slide power transmission device (540) and configured to amplify the stroke of the linear displacement of the slide power transmission device (540) in the direction of width; and
a slide mounting plate (570), a portion of the slide power transmission device (540) and the slide drive motor (530) being fixedly mounted to the slide mounting plate (570), and rigid multi-stage rails (571) being mounted on two sides of the slide mounting plate (570) in a direction of length of the incubator body (100), wherein the multi-stage rails (571) are configured to, by means of stages of rails partially sliding out relative to each other, support the culture vessel carrier mounting plate (560) that fully extends out after the stroke is amplified.

2. The culture vessel active-oscillation slide mechanism (500) of claim 1, **characterized in that**
the slide power transmission device (540) is of a lead screw structure that has a lead screw (541) and a lead screw nut (542) screwed onto the lead screw (541),
the lead screw (541) is connected to the output shaft (531) of the slide drive motor (530) via the coupling (543), and
the lead screw (541) of the slide power transmission device (540) and the coupling (543) are fixedly mounted to the slide mounting plate (570).

3. The culture vessel active-oscillation slide mechanism (500) of claim 2, **characterized in that**
the slide stroke amplification mechanism (550) is a multi-connecting-rod mechanism composed of a first multi-section connecting rod (552) and a second multi-section connecting rod (553), and
the slide stroke amplification mechanism (550) comprises:
a fixing portion, which is formed by a portion of the first multi-section connecting rod (552) and a portion of the second multi-section connecting rod (553) connected to each other and fixed to the coupling (543), and which is a portion that does not displace;
a stroke amplification portion, which is formed by another portion of the first multi-section connecting rod (552) and another portion of the second multi-section connecting rod (553) connected to each other, and to which a carrier mounting plate connection portion (551) for mounting the culture vessel carrier mounting plate (560) is fixed; and
a fulcrum portion, which is formed by a further portion of the first multi-section connecting rod (552) and a further portion of the second multi-section connecting rod (553) connected to each other and fixed to the lead screw nut (542), and which is capable of performing linear displacement in the direction of width and serves as a rotating fulcrum that enables the entire slide stroke amplification mechanism (550) to amplify the stroke of the linear displacement.

4. The culture vessel active-oscillation slide mechanism (500) of claim 3, **characterized in that**
the slide power transmission device (540) is arranged, along the direction of width of the incubator body (100), at a middle position of the slide mounting plate (570) in the direction of length, and
the fixing portion, the fulcrum portion and the stroke amplification portion are in a straight line.

5. The culture vessel active-oscillation slide mechanism (500) of claim 4, **characterized in that**
a distance between the stroke amplification portion and the fulcrum portion in the direction of width is greater than or equal to a distance between the fulcrum portion and the fixing portion in the direction of width.

6. The culture vessel active-oscillation slide mechanism (500) of any one of claims 3 to 5, **characterized in that**
the first multi-section connecting rod (552) is a three-section connecting rod composed of a first connecting rod, a second connecting rod and a third connecting rod connected in sequence by means of fasteners,
the second multi-section connecting rod (553) is a three-section connecting rod composed of a first connecting rod, a second connecting rod and a third connecting rod connected in sequence by means of fasteners,
one end of the first connecting rod of the first multi-section connecting rod (552) and one end of the first connecting rod of the second multi-section connecting rod (553) constitute the fixing portion,
one end of the third connecting rod of the first multi-section connecting rod (552) and one end of the third connecting rod of the second multi-section connecting rod (553) constitute the stroke amplification portion, and
the second connecting rod of the first multi-section connecting rod (552) and the second connecting rod of the second multi-section connecting rod (553) constitute the fulcrum portion in the middle.

7. The culture vessel active-oscillation slide mechanism (500) of claim 6, **characterized in that**
in the direction of length, a connecting portion between the first connecting rod and the second connecting rod and a connecting portion between the third connecting rod and the second connecting rod of each of the first multi-section connecting rod (552) and the second multi-section connecting rod (553) are close to the multi-stage rails (571) on the two sides of the slide mounting plate (570).

8. The culture vessel active-oscillation slide mechanism (500) of claim 1, **characterized in that**
the multi-stage rails (571) located inside the slide protective housing (510) and on the two sides of the slide mounting plate (570) each have a fixed rail (571a) and two stages of sliding rails, namely a first-stage sliding rail (571b) and a second-stage sliding rail (571c), and as the culture vessel carrier mounting plate (560) extends out, the culture vessel carrier mounting plate (560) is supported by the second-stage sliding rail (571c) in such a manner that the first-stage sliding rail (571b) partially slides out relative to the fixed rail (571a) and the second-stage sliding rail (571c) partially slides out relative to the first-stage sliding rail (571b).

9. The culture vessel active-oscillation slide mechanism (500) of claim 8, **characterized in that**
the first-stage sliding rail (571b) slides out no more than half relative to the fixed rail (571a), and
the second-stage sliding rail (571c) slides out no more than half relative to the first-stage sliding rail (571b).

10. The culture vessel active-oscillation slide mechanism (500) of claim 1, **characterized in that**
the culture vessel active-oscillation slide mechanism (500) further comprises a main oscillating motor (520), the main oscillating motor (520) being fixedly mounted to the slide mounting plate (570) and configured to enable the culture vessel active-oscillation slide mechanism (500) to actively oscillate.

11. The culture vessel active-oscillation slide mechanism (500) of claim 10, **characterized in that**
an oscillation driving shaft portion (572) and a plurality of oscillation driven shaft portions (573) are provided below the slide mounting plate (570),
each of the oscillation driving shaft portion (572) and the oscillation driven shaft portions (573) comprises:
a bearing pedestal fixed below the slide mounting plate (570);
a rolling bearing arranged in the respective bearing pedestal; and
an eccentric shaft that is eccentric relative to the respective rolling bearing,
the eccentric shaft of the oscillation driving shaft portion (572) is connected to the eccentric shaft of each of the oscillation driven shaft portions (573) via a belt pulley, and
the eccentric shaft (572c) of the oscillation driving shaft portion (572) is connected to an output shaft of the main oscillating motor (520).

12. A shaker incubator (10), comprising an incubator body (100), wherein an automatic seal door assembly (110) is provided on a front side of the incubator body (100), the automatic seal door assembly (110) has an automatic door (111) capable of being opened and closed automatically based on system control, and
a culture vessel carrier assembly (400) having a carrying plate (410) and a culture vessel (420) carried on the carrying plate (410), and an intelligent integrated control system unit (300) configured to control actions of automated components in the shaker incubator (10) are provided inside the incubator body (100),
**characterized in that**
a culture vessel active-oscillation slide mechanism (500) of any one of claims 1 to 11 is further provided inside the incubator body (100), the culture vessel active-oscillation slide mechanism (500) enables the culture vessel carrier assembly (400) to automatically slide out relative to the incubator body (100) and fully extend out after the automatic door (111) of the automatic seal door assembly (110) is opened, and enables the culture vessel carrier assembly (400) to automatically slide into the incubator body (100) before the automatic door (111) of the automatic seal door assembly (110) is closed and to actively oscillate after the automatic door (111) of the automatic seal door assembly (110) is closed.

13. The shaker incubator (10) of claim 12, **characterized in that**
the automatic seal door assembly (110) and the culture vessel active-oscillation slide mechanism (500) are provided as the automated components.
